# EUROPEAN PATENT APPLICATION

(11) **EP 2 460 890 A1**
(43) Date of publication of application: **06.06.2012**
(21) Application number: 10193362.0
(22) Date of filing: 01.12.2010
(51) Int. Cl.: C12Q 1/68

(54) **Gpnmb/Osteoactivin as a drug target and biomarker in cardiac diseases**

(71) Applicant: Max-Delbrück-Centrum für Molekulare Medizin (MDC), 13125 Berlin (DE)
(72) Inventor: Bader, Michael, 13125 Berlin (DE); Öczelik, Cemil, 13465 Berlin (DE); Mühlstedt, Silke, 10407 Berlin (DE)
(74) Representative: Krauss, Jan

(57) **Abstract**

The present invention relates to the gene Gpnmb and its use as a genetic marker in the incidence of cardiovascular conditions and cardiac diseases, such as complications derived from myocardial infarction. Gpnmb provides a valuable tool both for diagnostic as well as therapeutic approaches, in order to treat or prevent cardiovascular conditions and cardiac diseases, in particular complications derived from myocardial infarction.

SEQUENCE LISTING

<110>  Max-Delbrück-Centrum für Molekulare Medizin (MDC)
       Berlin - Buch

<120>  Gpnmb/Osteoactivin as a drug target and biomarker in cardiac
       diseases

<130>  FB24032

<160>  3

<170>  PatentIn version 3.5

<210>  1
<211>  572
<212>  PRT
<213>  Homo sapiens

<400>  1

Met Glu Cys Leu Tyr Tyr Phe Leu Gly Phe Leu Leu Leu Ala Ala Arg
1               5                   10                  15

Leu Pro Leu Asp Ala Ala Lys Arg Phe His Asp Val Leu Gly Asn Glu
            20                  25                  30

Arg Pro Ser Ala Tyr Met Arg Glu His Asn Gln Leu Asn Gly Trp Ser
        35                  40                  45

Ser Asp Glu Asn Asp Trp Asn Glu Lys Leu Tyr Pro Val Trp Lys Arg
    50                  55                  60

Gly Asp Met Arg Trp Lys Asn Ser Trp Lys Gly Gly Arg Val Gln Ala
65                  70                  75                  80

Val Leu Thr Ser Asp Ser Pro Ala Leu Val Gly Ser Asn Ile Thr Phe
                85                  90                  95

Ala Val Asn Leu Ile Phe Pro Arg Cys Gln Lys Glu Asp Ala Asn Gly
            100                 105                 110

Asn Ile Val Tyr Glu Lys Asn Cys Arg Asn Glu Ala Gly Leu Ser Ala
        115                 120                 125

Asp Pro Tyr Val Tyr Asn Trp Thr Ala Trp Ser Glu Asp Ser Asp Gly
    130                 135                 140

Glu Asn Gly Thr Gly Gln Ser His His Asn Val Phe Pro Asp Gly Lys
145                 150                 155                 160

Pro Phe Pro His His Pro Gly Trp Arg Arg Trp Asn Phe Ile Tyr Val
                165                 170                 175

Phe His Thr Leu Gly Gln Tyr Phe Gln Lys Leu Gly Arg Cys Ser Val
            180                 185                 190

Arg Val Ser Val Asn Thr Ala Asn Val Thr Leu Gly Pro Gln Leu Met
        195                 200                 205

Glu Val Thr Val Tyr Arg Arg His Gly Arg Ala Tyr Val Pro Ile Ala
    210                 215                 220

Gln Val Lys Asp Val Tyr Val Val Thr Asp Gln Ile Pro Val Phe Val
225                 230                 235                 240

Thr Met Phe Gln Lys Asn Asp Arg Asn Ser Ser Asp Glu Thr Phe Leu
                245                 250                 255

Lys Asp Leu Pro Ile Met Phe Asp Val Leu Ile His Asp Pro Ser His
            260                 265                 270

Phe Leu Asn Tyr Ser Thr Ile Asn Tyr Lys Trp Ser Phe Gly Asp Asn
        275                 280                 285

Thr Gly Leu Phe Val Ser Thr Asn His Thr Val Asn His Thr Tyr Val
    290                 295                 300

Leu Asn Gly Thr Phe Ser Leu Asn Leu Thr Val Lys Ala Ala Ala Pro
305                 310                 315                 320

Gly Pro Cys Pro Pro Pro Pro Pro Pro Pro Arg Pro Ser Lys Pro Thr
                325                 330                 335

Pro Ser Leu Ala Thr Thr Leu Lys Ser Tyr Asp Ser Asn Thr Pro Gly
            340                 345                 350

Pro Ala Gly Asp Asn Pro Leu Glu Leu Ser Arg Ile Pro Asp Glu Asn
        355                 360                 365

Cys Gln Ile Asn Arg Tyr Gly His Phe Gln Ala Thr Ile Thr Ile Val
    370                 375                 380

Glu Gly Ile Leu Glu Val Asn Ile Ile Gln Met Thr Asp Val Leu Met
385                 390                 395                 400

Pro Val Pro Trp Pro Glu Ser Ser Leu Ile Asp Phe Val Val Thr Cys
                405                 410                 415

Gln Gly Ser Ile Pro Thr Glu Val Cys Thr Ile Ile Ser Asp Pro Thr

420                 425                 430

Cys Glu Ile Thr Gln Asn Thr Val Cys Ser Pro Val Asp Val Asp Glu
        435                 440                 445

Met Cys Leu Leu Thr Val Arg Arg Thr Phe Asn Gly Ser Gly Thr Tyr
    450                 455                 460

Cys Val Asn Leu Thr Leu Gly Asp Asp Thr Ser Leu Ala Leu Thr Ser
465                 470                 475                 480

Thr Leu Ile Ser Val Pro Asp Arg Asp Pro Ala Ser Pro Leu Arg Met
                485                 490                 495

Ala Asn Ser Ala Leu Ile Ser Val Gly Cys Leu Ala Ile Phe Val Thr
            500                 505                 510

Val Ile Ser Leu Leu Val Tyr Lys Lys His Lys Glu Tyr Asn Pro Ile
        515                 520                 525

Glu Asn Ser Pro Gly Asn Val Val Arg Ser Lys Gly Leu Ser Val Phe
    530                 535                 540

Leu Asn Arg Ala Lys Ala Val Phe Phe Pro Gly Asn Gln Glu Lys Asp
545                 550                 555                 560

Pro Leu Leu Lys Asn Gln Glu Phe Lys Gly Val Ser
                565                 570

<210>  2
<211>  2775
<212>  DNA
<213>  Homo sapiens

<400>  2
tgccgcttaa taccatcaca tgatcctccc cgaggccctg tatttaatta aaatagagag      60

ggaggcacca cagatgccag aagaacactg ttgctcttgg tggacgggcc cagaggaatt     120

cagagttaaa ccttgagtgc ctgcgtccgt gagaattcag catggaatgt ctctactatt     180

tcctgggatt tctgctcctg gctgcaagat tgccacttga tgccgccaaa cgatttcatg     240

atgtgctggg caatgaaaga ccttctgctt acatgaggga gcacaatcaa ttaaatggct     300

ggtcttctga tgaaaatgac tggaatgaaa aactctaccc agtgtggaag cggggagaca     360

tgaggtggaa aaactcctgg aagggaggcc gtgtgcaggc ggtcctgacc agtgactcac     420

cagccctcgt gggctcaaat ataacatttg cggtgaacct gatattccct agatgccaaa     480

aggaagatgc caatggcaac atagtctatg agaagaactg cagaaatgag gctggtttat     540

ctgctgatcc gtatgtttac aactggacag catggtcaga ggacagtgac ggggaaaatg 600

gcaccggcca aagccatcat aacgtcttcc ctgatgggaa accttttcct caccaccccg 660

gatggagaag atggaatttc atctacgtct tccacacact tggtcagtat ttccagaaat 720

tgggacgatg ttcagtgaga gtttctgtga acacagccaa tgtgacactt gggcctcaac 780

tcatggaagt gactgtctac agaagacatg gacgggcata tgttcccatc gcacaagtga 840

aagatgtgta cgtggtaaca gatcagattc ctgtgtttgt gactatgttc cagaagaacg 900

atcgaaattc atccgacgaa accttcctca aagatctccc cattatgttt gatgtcctga 960

ttcatgatcc tagccacttc ctcaattatt ctaccattaa ctacaagtgg agcttcgggg 1020

ataatactgg cctgtttgtt tccaccaatc atactgtgaa tcacacgtat gtgctcaatg 1080

gaaccttcag ccttaacctc actgtgaaag ctgcagcacc aggaccttgt ccgccaccgc 1140

caccaccacc cagaccttca aaacccaccc cttctttagc aactactcta aaatcttatg 1200

attcaaacac cccaggacct gctggtgaca accccctgga gctgagtagg attcctgatg 1260

aaaactgcca gattaacaga tatggccact ttcaagccac catcacaatt gtagagggaa 1320

tcttagaggt taacatcatc cagatgacag acgtcctgat gccggtgcca tggcctgaaa 1380

gctccctaat agactttgtc gtgacctgcc aagggagcat tcccacggag gtctgtacca 1440

tcatttctga ccccacctgc gagatcaccc agaacacagt ctgcagccct gtggatgtgg 1500

atgagatgtg tctgctgact gtgagacgaa ccttcaatgg gtctgggacg tactgtgtga 1560

acctcaccct gggggatgac acaagcctgg ctctcacgag caccctgatt tctgttcctg 1620

acagagaccc agcctcgcct ttaaggatgg caaacagtgc cctgatctcc gttggctgct 1680

tggccatatt tgtcactgtg atctccctct tggtgtacaa aaaacacaag gaatacaacc 1740

caatagaaaa tagtcctggg aatgtggtca gaagcaaagg cctgagtgtc tttctcaacc 1800

gtgcaaaagc cgtgttcttc ccgggaaacc aggaaaagga tccgctactc aaaaaccaag 1860

aatttaaagg agtttcttaa atttcgacct tgtttctgaa gctcactttt cagtgccatt 1920

gatgtgagat gtgctggagt ggctattaac ctttttttcc taaagattat tgttaaatag 1980

atattgtggt ttggggaagt tgaatttttt ataggttaaa tgtcatttta gagatgggga 2040

gagggattat actgcaggca gcttcagcca tgttgtgaaa ctgataaaag caacttagca 2100

aggcttcttt tcattatttt ttatgtttca cttataaagt cttaggtaac tagtaggata 2160

gaaacactgt gtcccgagag taaggagaga agctactatt gattagagcc taacccaggt 2220

taactgcaag aagaggcggg atactttcag ctttccatgt aactgtatgc ataaagccaa 2280

tgtagtccag tttctaagat catgttccaa gctaactgaa tcccacttca atacacactc 2340

atgaactcct gatggaacaa taacaggccc aagcctgtgg tatgatgtgc acacttgcta 2400

gactcagaaa aaatactact ctcataaatg ggtgggagta ttttggtgac aacctacttt 2460

gcttggctga gtgaaggaat gatattcata tattcattta ttccatggac atttagttag     2520

tgctttttat ataccaggca tgatgctgag tgacactctt gtgtatattt ccaaattttt     2580

gtacagtcgc tgcacatatt tgaaatcata tattaagact ttccaaagat gaggtccctg     2640

gtttttcatg gcaacttgat cagtaaggat ttcacctctg tttgtaacta aaaccatcta     2700

ctatatgtta gacatgacat tctttttctc tccttcctga aaaataaagt gtgggaagag     2760

acaagaaaaa aaaaa                                                      2775

<210>  3
<211>  560
<212>  PRT
<213>  Homo sapiens

<400>  3

Met Glu Cys Leu Tyr Tyr Phe Leu Gly Phe Leu Leu Leu Ala Ala Arg
1               5                   10                  15

Leu Pro Leu Asp Ala Ala Lys Arg Phe His Asp Val Leu Gly Asn Glu
            20                  25                  30

Arg Pro Ser Ala Tyr Met Arg Glu His Asn Gln Leu Asn Gly Trp Ser
        35                  40                  45

Ser Asp Glu Asn Asp Trp Asn Glu Lys Leu Tyr Pro Val Trp Lys Arg
    50                  55                  60

Gly Asp Met Arg Trp Lys Asn Ser Trp Lys Gly Gly Arg Val Gln Ala
65                  70                  75                  80

Val Leu Thr Ser Asp Ser Pro Ala Leu Val Gly Ser Asn Ile Thr Phe
                85                  90                  95

Ala Val Asn Leu Ile Phe Pro Arg Cys Gln Lys Glu Asp Ala Asn Gly
            100                 105                 110

Asn Ile Val Tyr Glu Lys Asn Cys Arg Asn Glu Ala Gly Leu Ser Ala
        115                 120                 125

Asp Pro Tyr Val Tyr Asn Trp Thr Ala Trp Ser Glu Asp Ser Asp Gly
    130                 135                 140

Glu Asn Gly Thr Gly Gln Ser His His Asn Val Phe Pro Asp Gly Lys
145                 150                 155                 160

Pro Phe Pro His His Pro Gly Trp Arg Arg Trp Asn Phe Ile Tyr Val
                165                 170                 175

Phe His Thr Leu Gly Gln Tyr Phe Gln Lys Leu Gly Arg Cys Ser Val
            180                 185                 190

Arg Val Ser Val Asn Thr Ala Asn Val Thr Leu Gly Pro Gln Leu Met
        195                 200                 205

Glu Val Thr Val Tyr Arg Arg His Gly Arg Ala Tyr Val Pro Ile Ala
    210                 215                 220

Gln Val Lys Asp Val Tyr Val Val Thr Asp Gln Ile Pro Val Phe Val
225                 230                 235                 240

Thr Met Phe Gln Lys Asn Asp Arg Asn Ser Ser Asp Glu Thr Phe Leu
                245                 250                 255

Lys Asp Leu Pro Ile Met Phe Asp Val Leu Ile His Asp Pro Ser His
            260                 265                 270

Phe Leu Asn Tyr Ser Thr Ile Asn Tyr Lys Trp Ser Phe Gly Asp Asn
        275                 280                 285

Thr Gly Leu Phe Val Ser Thr Asn His Thr Val Asn His Thr Tyr Val
    290                 295                 300

Leu Asn Gly Thr Phe Ser Leu Asn Leu Thr Val Lys Ala Ala Ala Pro
305                 310                 315                 320

Gly Pro Cys Pro Pro Pro Pro Pro Pro Pro Arg Pro Ser Lys Pro Thr
                325                 330                 335

Pro Ser Leu Gly Pro Ala Gly Asp Asn Pro Leu Glu Leu Ser Arg Ile
            340                 345                 350

Pro Asp Glu Asn Cys Gln Ile Asn Arg Tyr Gly His Phe Gln Ala Thr
        355                 360                 365

Ile Thr Ile Val Glu Gly Ile Leu Glu Val Asn Ile Ile Gln Met Thr
    370                 375                 380

Asp Val Leu Met Pro Val Pro Trp Pro Glu Ser Ser Leu Ile Asp Phe
385                 390                 395                 400

Val Val Thr Cys Gln Gly Ser Ile Pro Thr Glu Val Cys Thr Ile Ile
                405                 410                 415

Ser Asp Pro Thr Cys Glu Ile Thr Gln Asn Thr Val Cys Ser Pro Val

420                 425                 430

Asp Val Asp Glu Met Cys Leu Leu Thr Val Arg Arg Thr Phe Asn Gly
        435                 440                 445

Ser Gly Thr Tyr Cys Val Asn Leu Thr Leu Gly Asp Asp Thr Ser Leu
    450                 455                 460

Ala Leu Thr Ser Thr Leu Ile Ser Val Pro Asp Arg Asp Pro Ala Ser
465                 470                 475                 480

Pro Leu Arg Met Ala Asn Ser Ala Leu Ile Ser Val Gly Cys Leu Ala
                485                 490                 495

Ile Phe Val Thr Val Ile Ser Leu Leu Val Tyr Lys Lys His Lys Glu
            500                 505                 510

Tyr Asn Pro Ile Glu Asn Ser Pro Gly Asn Val Val Arg Ser Lys Gly
        515                 520                 525

Leu Ser Val Phe Leu Asn Arg Ala Lys Ala Val Phe Phe Pro Gly Asn
    530                 535                 540

Gln Glu Lys Asp Pro Leu Leu Lys Asn Gln Glu Phe Lys Gly Val Ser
545                 550                 555                 560

## Description

The present invention relates to the gene Gpnmb and its use as a genetic marker in the incidence of cardiovascular conditions and cardiac diseases, such as complications derived from myocardial infarction. Gpnmb provides a valuable tool both for diagnostic as well as therapeutic approaches, in order to treat or prevent cardiovascular conditions and cardiac diseases, in particular complications derived from myocardial infarction.

### Description

Heart attacks are the leading cause of death for both men and women worldwide.

Myocardial infarction (MI) or acute myocardial infarction (AMI), commonly known as a heart attack, is the interruption of blood supply to a part of the heart, causing heart cells to die. This is most commonly due to occlusion (blockage) of a coronary artery following the rupture of a vulnerable atherosclerotic plaque, which is an unstable collection of lipids (fatty acids) and white blood cells (especially macrophages) in the wall of an artery. The resulting ischemia (restriction in blood supply) and oxygen shortage, if left untreated for a sufficient period of time, can cause damage or death (*infarction*) of heart muscle tissue (*myocardium*).

Acute complications of myocardial infarction include arrhythmia and cardiogenic shock, whereas chronic complications involve ventricular aneurysm and congestive heart failure.

As mentioned, myocardial infarction also leads to scar formation and subsequent reduced cardiac performance. The ultimate therapy after myocardial infarction would pursue stem cell-based regeneration. The aim of stem cell-mediated cardiac repair embodies restoration of cardiac function by regeneration of healthy myocardial tissue, which is accomplished by neo-angiogenesis and cardiogenesis. A major reservoir of adult autologous stem cells distal from the heart is the bone marrow. Adequate regulation of signaling between the bone marrow, the peripheral circulation and the infarcted myocardium is important in orchestrating the process of mobilization, homing, incorporation, survival, proliferation and differentiation of stem cells, that leads to myocardial regeneration.

Vandervelde *et al.* (in Vandervelde et al., Signaling factors in stem cell-mediated repair of infarcted myocardium. J Mol Cell Cardiol. 2005 Aug;39(2):363-76) describe stem cell-mediated cardiac regeneration. The paper discusses key signaling factors, including cytokines, chemokines and growth factors, which are involved in orchestrating the stem cell driven repair process with a focus on signaling factors known for their mobilizing and chemotactic abilities (SDF-1, G-CSF, SCF, IL-8, VEGF), signaling factors that are expressed after myocardial infarction involved in the patho-physiological healing process (TNF-alpha, IL-8, IL-10, HIF-1alpha, VEGF, G-CSF) and signaling factors that are involved in cardiogenesis and neo-angiogenesis (VEGF, EPO, TGF-beta, HGF, HIF-1alpha, IL-8). The future therapeutic application and capacity of secreted factors to modulate tissue repair after myocardial infarction is also reviewed.

Furthermore, Brunner et al (in: Brunner S, Engelmann MG, Franz WM Stem cell mobilisation for myocardial repair Expert Opin Biol Ther. 2008 Nov;8(11):1675-90) discuss the idea that autologous bone marrow derived stem cells (BMCs) can transdifferentiate into cardiomyocytes or vascular cells. They review summarizes conditions for stem cell mobilization, their use for therapeutic approaches to prevent ischemic cardiomyopathy after acute myocardial infarction and current clinical trials. Mechanisms for mobilization and homing of BMCs are discussed. The improvement in cardiac function after migration of autologous BMCs to the heart can be explained by their paracrine effects, inducing angiogenesis and preventing ischemic myocardium from apoptosis. These effects may explain why the number of circulating BMCs is directly correlated with cardiovascular risk and life expectancy.

Despite recent scientific progresses as discussed above, valuable and practical tools both for diagnostic as well as therapeutic approaches in order to treat or prevent cardiovascular conditions are still rare. It is therefore an object of the present invention, to provide novel genetic factors and markers, and in particular stem cell homing factors that are involved in the response to cardiovascular conditions and cardiac diseases, such as myocardial infarction. These markers would provide valuable tools both for diagnostic as well as therapeutic approaches in order to treat or prevent cardiovascular conditions and cardiac diseases, such as myocardial infarction and complications related thereto.

According to a first aspect thereof, the object of the present invention is solved by providing a method for determining a cardiovascular disease or condition in a subject, said method comprising: a) measuring an amount of a Gpnmb gene or gene product (in the context of the present invention used synonymously with the term "osteoactivin gene or gene product") in a biological sample derived from said subject, wherein said Gpnmb gene or gene product is: i) a protein comprising SEQ ID NO: 1 or 3; ii) a DNA encoding for a protein according to i), for example, corresponding to SEQ ID NO: 2, or a nucleic acid derived therefrom; iii) a nucleic acid comprising a sequence hybridizable to a DNA according to ii), for example SEQ ID NO: 2, or a complement under conditions of high stringency, or a protein comprising a sequence encoded by said hybridizable sequence; iv) a nucleic acid at least 90% homologous to a DNA according to ii), for example SEQ ID NO: 2, or a complement; or a protein encoded thereby; and b) comparing the amount of said Gpnmb gene product in said subject with the amount of Gpnmb gene product present in a non-cardiovascular disease biological sample, preferably a non-ischemic cardiac tissue sample or predetermined standard for a non-cardiovascular disease biological sample, preferably a non-ischemic cardiac tissue sample, wherein an elevated amount of said Gpnmb gene product in the subject compared to the amount in the non-ischemic cardiac tissue sample or pre-determined standard for a non-ischemic cardiac tissue sample indicates a cardiovascular disease or condition in said subject.

Preferably, the non-cardiovascular disease biological sample is a biological sample derived from a healthy subject, that is, a subject having no or having no history of a cardiovascular disease or condition. More preferably, the non-cardiovascular disease biological sample can be a sample from a subject having no or having no history of mycordial infarction.

Preferred is a method according to the present invention, wherein said determining comprises a diagnosis or prognosis of a cardiovascular disease or condition selected from the group consisting of myocardial infarction, arrhythmia, cardiogenic shock, ventricular aneurysm, congestive heart failure, ischemic heart disease, and other related conditions related to or resulting from an ischemic event.

Another important aspect of the present invention then relates to a method for identifying a compound that is interacting with a Gpnmb gene or gene product (in the following also designated as "GPNMB peptide") selected from: i) a protein comprising SEQ ID NO: 1 or 3; ii) a DNA encoding for a protein according to i), for example, corresponding to SEQ ID NO: 2, or a nucleic acid derived therefrom; iii) a nucleic acid comprising a sequence hybridizable to a DNA according to ii), for example SEQ ID NO: 2, or a complement under conditions of high stringency, or a protein comprising a sequence encoded by said hybridizable sequence; iv) a nucleic acid at least 90% homologous to a DNA according to ii), for example SEQ ID NO: 2, or a complement; or a protein encoded thereby, comprising the steps of: a) contacting said peptide with at least one potentially interacting compound, and b) detecting and/or measuring binding of said compound to said peptide.

The present invention thus generally relates to the use of the GPNMB gene or peptide as a diagnostic tool (marker) for cardiovascular diseases or conditions, and as a screening tool for agents and compounds for treating and/or preventing such cardiovascular diseases or conditions. Both the amino acid sequence (SEQ ID No. 1), and the respective nucleotide sequence (SEQ ID No. 2) of the human transmembrane glycoprotein NMB isoform a precursor can be found in the database at Accession number NP_001005340 (status of 12-NOV-2010). The amino acid sequence of the NMB isoform b precursor is shown in SEQ ID No. 3.

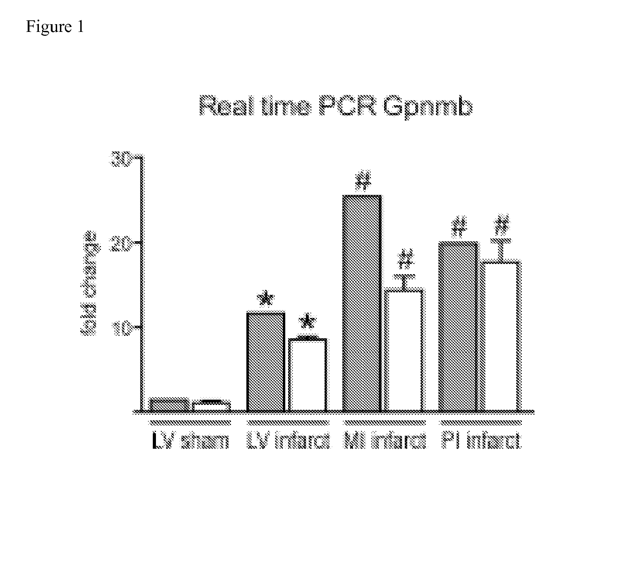

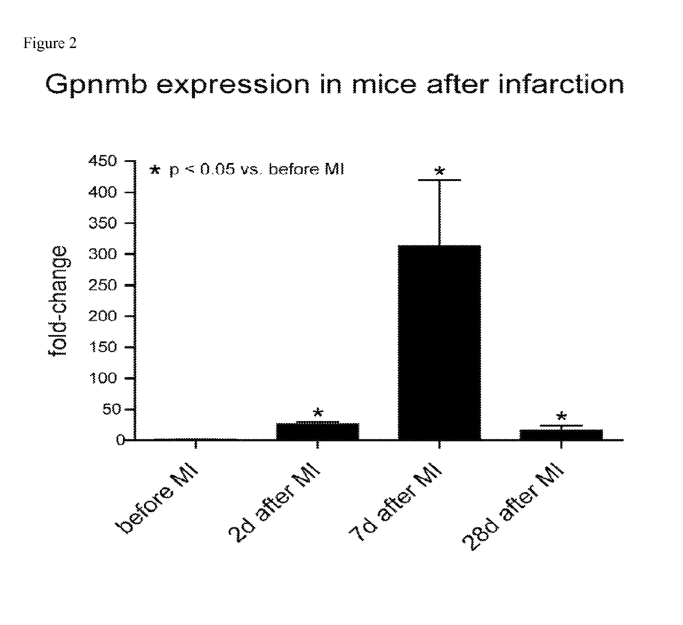

Surprisingly, a role of the GPNMB gene in the context of cardiovascular diseases or conditions, such as, for example, myocardial infarction, was identified during screening experiments of the inventors for (stem cell homing) factors involved in cardiac repair. GPNMB (Osteoactivin) was identified as a strongly induced gene after myocardial infarction (see Figures 1 and 2).

Until today, a connection between cardiovascular diseases or conditions and GPNMB (Osteoactivin) was unknown. GPNMB is a glycoprotein and was first described in 1995 (Weterman et al., Int J Cancer, 1995). GPNMB exists in two different isoforms, one is a transmembrane protein, the other one is secreted. Therefore, several biological functions are predicted for GPNMB, a receptor function, a ligand function, and enzymatic functions.

Abdelmagid *et al.* (in: Abdelmagid, S. M. et al., Osteoactivin, an anabolic factor that regulates osteoblast differentiation and function Exp. Cell Res. 2008) describe osteoactivin (OA) as a novel glycoprotein that is highly expressed during osteoblast differentiation. Using Western blot analysis, they show that OA protein has two isoforms, one is transmembranous and the other is secreted into the conditioned medium of primary osteoblasts cultures. Glycosylation studies showed that OA isoform of 115 kDa is highly glycosylated. The functional role of the secreted OA isoform was revealed when cultures treated with anti-OA antibody, showed decreased osteoblast differentiation compared to untreated control cultures. Gain-of-function in osteoblasts using the pBABE viral system showed that OA overexpression in osteoblast stimulated their differentiation and function. The availability of a naturally occurring mutant mouse with a truncated OA protein provided further evidence that OA is an important factor for terminal osteoblast differentiation and mineralization. Using bone marrow mesenchymal cells derived from OA mutant and wild-type mice and testing their ability to differentiate into osteoblasts showed that differentiation of OA mutant osteoblasts was significantly reduced compared to wild-type osteoblasts. Collectively, the data suggest that OA acts as a positive regulator of osteoblastogenesis.

Selim *et al.* (in: Selim et al., Anti-osteoactivin antibody inhibits osteoblast differentiation and function in vitro. Crit Rev Eukaryot Gene Expr, 2003) describe the osteoactivin (OA) cDNA as having an open reading frame of 1716 bp encoding a protein of 572 aa, the first 21 aa constitute a signal peptide. Using the Chariot transfection reagent as a vehicle to deliver anti-OA antibody into the cells, they demonstrated that anti-OA antibody significantly inhibited osteoblast differentiation markers, including alkaline phosphatase activity, nodule formation, osteocalcin production, and calcium deposition, without affecting cell proliferation or viability. These data suggest that OA is an osteoblast-related protein that plays an important role in the regulation of osteoblast differentiation and function.

Williams et al. (in: Williams MD, Esmaeli B, Soheili A, Simantov R, Gombos DS, Bedikian AY, Hwu P. GPNMB expression in uveal melanoma: a potential for targeted therapy. Melanoma Res. 2010 Jun;20(3):184-90) describe a high expression of glycoprotein-NMB (GPNMB) in cutaneous melanomas that led to the development of CDX-011 (glembatumumab vedotin), a fully human monoclonal antibody against the extracellular domain of GPNMB conjugated to the cytotoxic microtubule toxin monomethylauristatin E. Ongoing phase II trials suggest that CDX-011 has activity against advanced cutaneous melanomas.

Furthermore, several authors have described osteoactivin as a protein that is expressed in aggressive human breast cancers and is capable of promoting breast cancer metastasis to bone (Oganagai et al., J Hepatol, 2003; Tse et al., Clin Cancer Res, 2006; Kuan et al., Clin Cancer Res, 2006; Rose et al., Mol Cancer Res, 2007).

Finally, the transgenic overexpression of Gpnmb seems to attenuate hepatic fibrosis in rats (Abe et al., BBRC, 2007); Gpnmb might trigger renal interstitial fibrosis after unilateral ureteral obstruction (Nakamura et al., Exp Toxicol Pathol., 2007); and the overexpression of Gpnmb protects skeletal muscle from severe degeneration after long-term denervation (Furochi et al., J Med Invest., 2007). Therefore, Gpnmb may be important for protective and regenerative processes after injury in liver, kidney and skeletal muscle.

In view of this, possible applications of the present invention for humans include:
a) Diagnostic approaches: Since Gpnmb expression and activity increases during myocardial infarction, genetic tests can be developed to assess an individual risk for related conditions based on the induction, and to develop a personalized treatment plan as an intervention approach.
b) Pharmaceutical and therapeutic approaches: The inventors' data indicate that a modulator, and in particular an inhibitor of Gpnmb action can be used as a myocardial infarction drug in mammals/humans, and related aspects of vascular and cardiovascular diseases or conditions that are usually secondary to myocardial infarction.

The term "GPNMB gene" or "GPNMB peptide" shall further include homologs of the GPNMB gene or peptide in other mammalian organisms in addition to the human, such as, for example, mouse, rat, dog, cat, monkey, and rabbit. The term "homology" or "homologous" as used herein means a value obtained by a BLAST [Basic local alignment search tool; Altschul, S. F. et al., J. Mol. Biol., 215, 403-410, (1990)] search. The homology in the amino acid sequence may be calculated by a BLAST search algorithm. More particularly, it may be calculated using a bl2seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a Query sequence, and "BLOSUM62" as a Matrix are used, respectively.

Preferred is a diagnostic or therapeutic method according to the present invention, wherein said biological sample contains biological material that is derived from tissues or cells of the heart muscle, such as, for example, cells from the myocardial infarct region (MI) and/or the peri-infarct region (PI).

Both the expression and/or abundance of the GPNMB peptide or the mRNA thereof as well as the biological activity can be determined with any suitable assay known to the person of skill. Common assays involve PCR, Northern-, Western- or Southern-Blots, and/or enzymatic assays. Thus, preferred is a method according to the present invention, wherein said diagnosis comprises the use of GPNMB peptide-specific antibodies, hybridisation analysis, quantitative PCR, mRNA analysis, quantitative analysis of the amount of GPNMB peptide, sequencing of the GPNMB-locus, and/or an analysis of the activity of the GPNMB peptide in said sample.

The object of the present invention is also solved by a method for identifying a compound that is interacting with a GPNMB peptide according to the present invention, comprising screening for such compounds comprising the steps of: a) contacting said peptide with at least one potentially interacting compound, and b) detecting and/or measuring binding of said compound to said peptide.

Measuring of binding of the compound to GPNMB can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compo und or of the protein, e.g. a change of the redox properties of either GPNMB or the binding compound, upon binding. Suitable methods of detecting such changes comprise, for example, potent i-ometric methods. In a preferred embodiment, detection occurs by a method selected from the group comprising fluorimetry, surface plasmon resonance, gel filtration chromatography, mass spectrometry and nuclear magnetic resonance (NMR). Further methods for detecting and/or measuring the binding of the two components to each other are known in the art and can without limitation also be used to measure the binding of the potential interacting co m-pound to GPNMB or GPNMB fragments. The effect of the binding of the compound or the activity of GPNMB can also be measured indirectly, for example, by assaying an biological activity of GPNMB after binding.

This method is suitable for the determination of compounds that can interact with a GPNMB peptide and to identify, for example, inhibitors, activators, competitors or modulators of a GPNMB peptide, in particular inhibitors, activators, competitors or modulators of the biological activity(ies) of the GPNMB peptide. Another aspect is directed at compounds that modulate the expression of GPNMB in a cell/in cells. Inhibitors are preferred.

Such a modulator can be an antisense molecule or siRNA molecule that binds to the mRNA transcribed from the GPNMB gene and inhibits its translation into a functional protein. In particular, such siRNA molecules preferably have a length of 18-22 nucleotides and are reverse complementary in sequence to the GPNMB mRNA sequence. A suited siRNA molecule can be determined by a skilled artisan based on the information given herein together with his general knowledge.

Such a modulator can be transfected into a cell using standard cell culture techniques. E.g., lipofection, gene gun bombardment, electroporation, and/or virus infection (e.g. using lentiviruses) can be used.

The term "contacting" in the present invention means any interaction between the potentially binding substance(s) with a GPNMB peptide, whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip and GPNMB (or a functional part thereof) is subsequently contacted with such a chip.

The GPNMB employed in a method of the present invention can be a full length protein or a fragment with N/C-terminal and/or internal deletions. Preferably the fragment is either an N-terminal fragment comprising the enzymatic region of the polypeptide or a C-terminal fragment comprising the cytoplasmic region, depending on whether potentially interacting co m-pounds are sought that specifically interact with the N- or C-terminal fragment.

In a preferred embodiment of the method according to the present invention, determining an interaction between said candidate compound(s) and said GPNMB peptide comprises isolation of the complexes. Such isolation can be performed using common separation and/or purification techniques, such as chromatography, gel filtration, precipitation, immune absorption, gel electrophoresis, centrifugation, and the like. Furthermore preferred is method according to the present invention wherein said determining an interaction between said candidate compounds and said at least one complex comprises a detection of the complex using antibodies, radioactivity detection methods, dye detection methods, enzymatic detection methods and mass spectroscopy.

As mentioned above, in a preferred method according to the present invention screening is performed *in vivo* or in vitro. Preferred are uses *in vitro.*

In a preferred embodiment of the method according to the present invention, said method further comprises the steps of: a) selecting a binding compound, b) modifying the binding compound to generate a variety of modified binding compounds, c) contacting said peptide with each of the modified binding compounds, d) measuring binding of said modified compounds to said peptide, and e) optionally, repeating steps a) to d) for one or more times.

As a further step, after measuring the binding of a potentially interacting compound the compound can be selected, for example, on grounds of the measured binding activity or on grounds of the detected increase or decrease of GPNMB peptide activity and/or expression.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with a method of the present invention, i.e. they are contacted with a GPNMB peptide and subsequently binding of the modified compounds to the GPNMB peptide is measured. In this step, both the binding per se can be measured and/or the effect of the function of the GPNMB peptide like, e.g. the enzymatic activity of the polypeptide can be measured. If needed, the steps of selecting the binding compound, modifying the binding compound, contacting the binding compound with a GPNMB peptide and measuring the binding of the modified compounds to the protein can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity of the GPNMB peptide.

In a preferred embodiment of the method according to the present invention, said method then further comprises the step: f) measuring and/or detecting the biological function of said GPNMB peptide in the presence or absence of said binding compound as identified herein. As described above, several biological functions are predicted for GPNMB, a receptor (signaling) function, a ligand function, and enzymatic functions.

Yet another aspect of the present invention then relates to a method for identifying a compound modulating the expression and/or biological activity of a GPNMB peptide in a biological sample comprising cells, comprising the steps of a) contacting a cell expressing a GPNMB peptide with at least one potentially modulating compound, optionally one as identified according to the present invention as above, and b) measuring a modulation in the expression and/or biological activity of said GPNMB peptide in said cell. Thus, another aspect is directed at compounds that modulate the expression of GPNMB in a cell/in cells.

In one further aspect of the present invention, the screening is performed using a cell which is transformed with an expression vector comprising a polynucleotide encoding a GPNMB peptide, preferable according to SEQ ID NO: 1, and expresses said polypeptide. The cell can be a prokaryotic or eukaryotic cell, and the expression constructs can be present extrachromosomally or integrated into the chromosome. The polypeptide can be expressed in the form of a fusion protein, for example together with an enzymatically active moiety as reporter-construct, in order to be able to detect the expression product. Preferred host cells are derived from cancer cell lines, or cells selected from the heart or skeletal muscle, pancreas, kidney, and/or hypothalamus.

In another aspect of the method according to the present invention, the screening is performed using a non-human transgenic mammal overexpressing GPNMB peptide and/or carrying a GPNMB peptide genetic reporter-construct. Preferred is a transgenic mouse, rat, pig, goat or sheep, wherein the reporter-construct is preferably expressed in cells selected from the heart muscle, skeletal, pancreas, kidney, and/or hypothalamus of said animal. Methods to produce these non-human transgenic mammal overexpressing GPNMB peptide and/or carrying a GPNMB peptide genetic reporter-construct are well known to the person of skill in the art.

In another aspect of the method according to the present invention, contacting said protein with at least one potentially interacting compound is performed in the presence of at least one cofactor and/or coenzyme. A cofactor is a non-protein chemical compound that is bound to a protein and is required for the protein's biological activity. These proteins are commonly enzymes, and cofactors can be considered "helper molecules" that assist in biochemical transformations. Cofactors are either organic or inorganic. They can also be classified depending on how tightly they bind to an enzyme, with loosely-bound cofactors termed coenzymes and tightly-bound cofactors termed prosthetic groups. Coenzymes are small organic molecules that transport chemical groups from one enzyme to another.

Further preferred is a method according to the present invention, wherein the biological sample is selected from tissues or cells of the heart muscle, such as, for example, cells from the myocardial infarct region (MI) where scarring of the myocardium takes place and/or the peri-infarct region (PI), the area of the muscle immediately surrounding the infarct myocardium.

Further preferred is a method according to the present invention, wherein said compound as identified is an inhibitor of the expression and/or biological activity of said GPNMB peptide in said cell and/or tissue. Inhibiting the expression and/or biological activity of said GPNMB peptide in said cell and/or tissue shall include a reduction of the expression and/or biological activity up to a complete inhibition of the expression and/or biological activity of said GPNMB peptide in said cell and/or tissue.

Further preferred is a method according to the present invention, wherein said compound is selected from a peptide library, a combinatory library, a cell extract, in particular a plant cell extract, a "small molecular drug", an antibody or fragment thereof, an antisense oligonucleotide, an siRNA, a protein, and/or a protein fragment.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr Med Chem. 2002 Dec;9(23):2129-45, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it is necessary to optimize the natural product structure by chemical variation. Solid-phase chemistry is said to become an efficient tool for this optimization process, and recent advances in this field are highlighted in this review article. Other related references include Edwards PJ, Morrell AI. Solid-phase compound library synthesis in drug design and development. Curr Opin Drug Discov Devel. 2002 Jul;5(4):594-605.; Merlot C, Domine D, Church DJ. Fragment analysis in small molecule discovery. Curr Opin Drug Discov Devel. 2002 May;5(3):391-9. Review; Goodnow RA Jr. Current practices in generation of small molecule new leads. J Cell Biochem Suppl. 2001;Suppl 37:13-21; which describes that the current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures. Subsequent optimization of such compounds is often accelerated because of the structure-activity relationship (SAR) information encoded in these lead generation libraries. Lead optimization is often facilitated due to the ready applicability of high-throughput chemistry (HTC) methods for follow-up synthesis. Some of the strategies, trends, and critical issues central to the success of lead generation processes are discussed. One use of such a library is finally described in, for example, Wakeling AE, Barker AJ, Davies DH, Brown DS, Green LR, Cartlidge SA, Woodburn JR. Specific inhibition of epidermal growth factor receptor tyrosine kinase by 4-anilinoquinazolines. Breast Cancer Res Treat. 1996;38(1):67-73.

The library of the potentially interacting compounds contains entities that are usually "non-labeled", i.e. that do not contain a marker that would be necessary for an identification of the library compound. Nevertheless, also libraries of labeled potentially interacting compounds can be screened without using the labels of the compounds.

In one embodiment of the present invention, a method is provided, wherein the potentially interacting candidate compounds are selected from enzymes, polypeptides, peptides, antibodies and fragments thereof, nucleic acids or derivatives thereof, and chemical entities having a molecular mass of less than 1000 kDa ("small molecules"). Thus, in one library of the present invention potentially interacting compounds are provided that potentially interact with the GPNMP peptide to be analyzed (screened). Examples of such compounds are synthetic and/or naturally occurring chemical compounds, peptides, proteins, antibodies, and the like. Since the library is related to "small" compounds, i.e. other that complete enzymes, antibodies or other proteins, the molecular weight of these compounds is preferably below 1000 kDa, more preferably below 500 kDa. Such compounds can be suitable as "leads" for further optimization. One example of a peptide library is, for example, described in Sachpatzidis A, et al. Identification of allosteric peptide agonists of CXCR4. J Biol Chem 2003 Jan 10;278(2):896-907, wherein a synthetic cDNA library coding for 160,000 different SDF-based peptides was screened for small molecule CXCR4 agonist activity in a yeast strain.

In another embodiment of the method according to the present invention, said potentially interacting candidate compounds is a nucleic acid, such as a DNA, RNA and/or PNA. Such nucleic acids can be present in the form of oligonucleotides or polynucletides, covering specific binding-specific nucleotide specific nucleic acid sequences and/or motifs. Hybrid nucleic acids between the different forms might also be employed. Furthermore, the library can be present on a chip for high-throughput screening purposes.

Similar to the strategies for identifying compounds that interact with GPNMB, compounds can be identified that modulate the expression of GPNMB in a cell. In preferred strategies, the expression of GPNMB can be monitored using a reporter-construct for GPNMB (in order to analyze the translation efficiency and/or stability of the GPNMB peptide), for an example a fusion protein comprising a detectable fusion member (such as an enzymatic or fluorophoric group), or the amount of mRNA as present in a cell can be measured, for example, by Northern blot. The expression can also be analyzed and monitored by using chip-analysis or rtPCR. Preferred compounds that modulate the expression of GPNMB in a cell are selected from specific antisense oligonucleotide(s), or siRNA(s) for RNAi. Respective methods are well described in the literature and known to the person of skill.

As mentioned above, the library of potentially interacting candidate compounds can be present in different formats, such as in liquid solution, such as in tubes, microtiter-plates, or on a solid support, such as on filters, glass slides, silicon surfaces, beads or a customised chemical microarray. Microarrays are preferred due to their easy handling and their uses in high-throughput formats.

In one preferred embodiment of the method according to the present invention, the potentially interacting candidate compounds are bound to beads, such as sepharose (e.g. NHS-activated sepharose) or agarose beads. In contrast to Evans DM et al. (1996, see above), the present invention uses immobilized compound libraries that are screened with GPNMB peptide, which allows for a much more flexible screening procedure than using columns with immobilised binding partners. Furthermore, on columns the "in vivo-like conditions" of the present method is easily lost, which renders some interactions unspecific and leads to imprecise results. Preferably, said potentially interacting candidate compounds are bound to said beads via an amino-group or carboxy-group.

Further preferred is a method according to the present invention, wherein the diagnostic method is for monitoring the effect of therapy administered to a subject having a cardiovascular disease or condition. That is, since the Gpnmb expression and activity increases during myocardial infarction, genetic tests can assess an individual risk for related conditions based on the induction and the disease or condition, and to develop and follow a further (preferably personalized) treatment plan as an intervention approach.

In the context of the present invention, it is preferred that the subject to be diagnosed and/or treated is a mammal, such as, for example, a mouse, rat, dog, cat, monkey, rabbit, or human.

In a further embodiment of the method of the present invention, the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routes of application like, for example, intravenous solution, sprays, band-aids or pills.

Another aspect of the present invention relates to a method for manufacturing a pharmaceutical composition for treating or preventing a cardiovascular condition or disease, comprising the steps of: performing a screening method according to the present invention, and formulating said modulator as screened and identified into a pharmaceutical composition.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or non-solvent. In many cases, water or an organic liquid can be used.

Yet another aspect of the present invention is directed at a pharmaceutical composition for treating or preventing a cardiovascular condition or disease, obtainable by a method according to the invention as above. In certain embodiments, the compound (modulator) is administered to the subject by administering a recombinant nucleic acid. Preferably, the recombinant nucleic acid is a gene therapy vector.

Another aspect of the present invention then relates to a pharmaceutical composition for treating or preventing a cardiovascular condition or disease, which includes a compound that modulates the expression and/or biological activity of a GPNMB peptide, optionally in a cell (modulator). This modulator can be obtainable by a method according to the present invention. Preferred is a pharmaceutical composition according to the present invntion comprising a therapeutically effective amount of a compound that down-regulates and/or inhibits the expression and/or biological activity of GPNMB, and a pharmaceutically acceptable carrier.

Another aspect of the present invention relates to a method or use as above, wherein the pharmaceutical composition further comprises additional pharmaceutically active ingredients that modulate the cardiovascular condition or disease to be treated, such as a cardiovascular disease is selected from the group consisting of atherosclerosis, coronary artery disease, ischemic heart disease, myocardial infarction, chronic heart insufficiency, angina, stroke and other related conditions related to or resulting from an ischemic event

Yet another aspect of the present invention relates to a method for treating or preventing a cardiovascular disease, comprising administering to a subject in need thereof an effective amount of a pharmaceutical composition according to the present invention. Preferably, an inhibiting active agent is administered in form of a pharmaceutical composition, such as an antibody, antisense nucleotide or an inhibiting binding compound. Preferably, said patient is a human being. Treating is meant to include, e.g., preventing, treating, reducing the symptoms of, or curing the cardiovascular disease or condition.

An "effective amount" is an amount of the compound(s) as mentioned above that a) acts on the expression and/or abundance of GPNMB as analysed, and which alleviates symptoms as found for the cardiovascular disease or condition. Alleviating is meant to include, e.g., preventing, treating, reducing the symptoms of, or curing the cardiovascular disease or condition.

Another aspect of the present invention relates to a method for treating or preventing a cardiovascular disease or condition, comprising a diagnostic method according to the present invention as above, and providing a treatment to said mammal based on, at least in part, the result of said diagnosis. In general, the attending physician will base a treatment, and in particular the cardiovascular disease or condition-preventive measures, on the diagnostic data regarding GPNMB as analyzed in said subject (patient), wherein said patient diagnostic data regarding GPNMB can be integrated with other individual patient data (clinical data, family history, DNA, etc.), and a treatment can also be performed based on the combination of these factors. This method of the present invention for example involves integrating individual diagnostic data with patient clinical information and general healthcare statistics to enable, for example, the application of personalized medicine to the patient. Significant information about drug effectiveness, drug interactions, physiologic heart regularities and other patient status conditions could be correlated with the specific diagnostic data.

Another aspect of the present invention then relates to the use of a modulator of the expression and/or the biological activity of GPNMB in a cell, in particular to the use of a modulator as described above, for the manufacture of a pharmaceutical composition for treating or preventing cardiovascular diseases or conditions. Further preferred is a use according to the present invention, wherein said modulator is an inhibitor of the expression and/or biological activity of GPNMB. Such a modulator can be an antisense molecule or siRNA molecule that binds to the mRNA transcribed from the GPNMB gene and inhibits its translation into a functional protein.

Preferred is a use according to the present invention, wherein said cardiovascular disease or condition is selected from the group consisting of atherosclerosis, coronary artery disease, ischemic heart disease, myocardial infarction, chronic heart insufficiency, angina, stroke and other related conditions related to or resulting from an ischemic event.

The following figures, sequences, and examples merely serve to illustrate the invention and should not be construed to restrict the scope of the invention to the particular embodiments of the invention described in the examples. All references cited in the text are hereby incorporated in their entirety by reference.

The improvement of the cardiac function (fractional shortening, ejection fraction), as well as the remodeling (LVEDD, LVESD) after experimental induction of infarction in DBA/2J mice (mutated Gpnmb, lack of function) compared to DBA/2J mice (wild type Gpnmb) clearly establishes Gpnmb as a novel target for a therapeutic (modulation of the function/expression) intervention, and supports the present approaches for screening for modulators, and particularly inhibitors, of GPNMB.

The significant induction of Gpnmb/osteaoactivin after infarction in rat and mouse also clearly establish Gpnmb as a novel diagnostic marker.

SEQ ID NO 1 shows the amino acid sequence of the human GPNMB polypeptide isoform a precursor (Isoform 1).

SEQ ID NO 2 shows the nucleic acid sequence of the human GPNMB polypeptide according to SEQ ID NO 1.

SEQ ID NO 3 shows the amino acid sequence of the human GPNMB polypeptide isoform b precursor (Isoform 2).
Figure 1 shows the results for the real-time expression analysis for GPNMB in left ventricle (LV) sham, LV, myocardial infarct (MI) region and peri-infarct (PI) region.
Figure 2 shows the results for the real-time expression analysis for GPNMB over time after infarction.

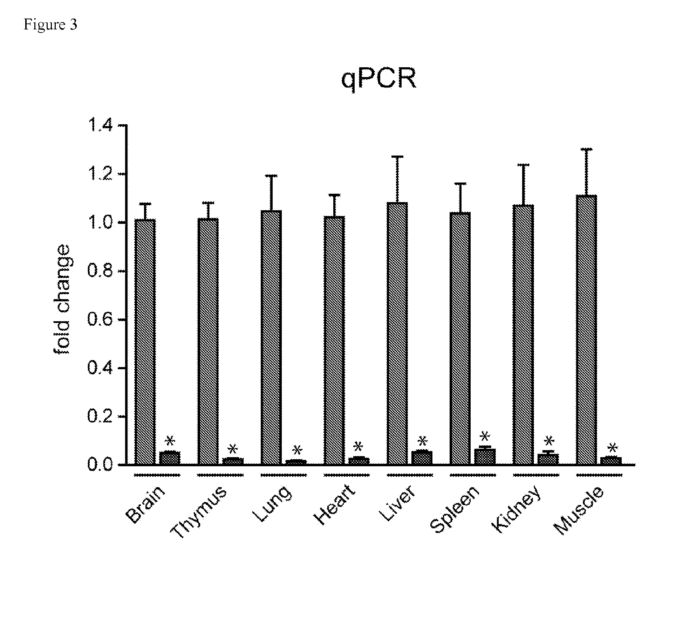

**Figure 3** shows the difference in expression of GPNMB in tissues of DBA/2J vs DBA/2J-GPNMB+ mice (left columns) according to example 2.

### Example 1:

**mRNA expression profiling of control vs. infarcted hearts and identification of GPNMB** In this experiment, mRNA expression profiling of control vs. infarcted hearts using microarray screening was performed. For this, myocardial infarction was induced in WKY rats through coronary artery ligation.

Then, mRNA isolated from the myocardial infarct region (MI), healthy left ventricle (LV) and peri-infarct region (PI) was studied in an expression profiling assay using the GeneChip® Rat Gene 1.0 ST Array (Affymetrix). Of the approx. 23000 genes as studied, >5000 genes were significantly regulated, >2700 genes were up-regulated in the peri-infarct region. 325 of the genes as analyzed encoded for secretory proteins, and 60 genes were up-regulated ≥1.5 fold.

Finally, 35 genes were selected to be tested via RT Real Time PCR. Out of these, gpnmb/osteoactivin was established as the most promising candidate, and selected for further analysis (see Figures 1 and 2).

### Example 2:

### Functional characterization of Gpnmb in DBA/2J mice

DBA/2J mice have a mutant *Gpnmb* gene (R150X premature stop codon) and they develop a form of glaucoma preceded by a pigment dispersing iris disease and abnormalities of the immunosuppressive ocular microenvironment (Anderson et al., BMC Genet, 2008).

These mice were used together with DBA/2J-GPNMD+ mice in order to study the effect of GPNMD loss of function on myocardial infarction. The mice were examined by echo before coronary artery ligation, by echo 1 week after coronary artery ligation, and both by echo and MRI 4 weeks after coronary artery ligation.

The cardiac function (fractional shortening, ejection fraction), as well as the remodeling (Left Ventricular End Diastolic Diameter (LVEDD), Left Ventricular End Systolic Diameter (LVESD)) after experimental induction of infarction in the DBA/2J mice (mutated Gpnmb, lack of function) was clearly improved when compared to the DBA/2J mice (wild type Gpnmb).

The features disclosed in the foregoing description, in the claims and/or in the accompanying drawings may, both separately and in any combination thereof, be material for realising the invention in diverse forms thereof.

## Claims

1. A method for determining a cardiovascular disease in a subject, said method comprising:
a) measuring an amount of a Gpnmb gene or gene product in a biological sample derived from said subject, wherein said Gpnmb gene or gene product is:
i) a protein comprising SEQ ID NO: 1 or 3;
ii) a DNA encoding for a protein according to i), for example, corresponding to SEQ ID NO: 2, or a nucleic acid derived therefrom;
iii) a nucleic acid comprising a sequence hybridizable to a DNA encoding for a protein according to i), for example, corresponding to SEQ ID NO: 2, or its complement under conditions of high stringency, or a protein comprising a sequence encoded by said hybridizable sequence;
iv) a nucleic acid at least 90% homologous to a nucleic acid according to ii), for example SEQ ID NO: 2, or a complement; or a protein encoded thereby; and
b) comparing the amount of said Gpnmb gene product in said subject with the amount of Gpnmb gene product present in a non-ischemic cardiac tissue sample or predetermined standard for a nonischemic cardiac tissue sample,
wherein an elevated amount of said Gpnmb gene product in the subject compared to the amount in a non-cardiovascular disease biological sample or pre-determined standard for a non-cardiovascular disease biological sample indicates a cardiovascular disease in said subject.

2. The method according to claim 1, wherein said determining comprises a diagnosis or prognosis of a cardiovascular disease selected from the group consisting of myocardial infarction, arrhythmia, cardiogenic shock, ventricular aneurysm, congestive heart failure, ischemic heart disease, and other related conditions related to or resulting from an ischemic event.

3. A method for identifying a compound that is interacting with a GPNMB peptide according to claim 1, comprising the steps of:
a) contacting said peptide with at least one potentially interacting compound, and
b) detecting and/or measuring binding of said compound to said peptide, and optionally
c) selecting a binding compound,
d) modifying the binding compound to generate a variety of modified binding compounds,
e) contacting said peptide with each of the modified binding compounds,
f) measuring binding of said modified compounds to said peptide, and
g) optionally, repeating steps c) to g) for one or more times.

4. The method according to claim 3, further comprising the step of:
f) measuring and/or detecting the biological function of said peptide according to claim 1 in the presence or absence of said binding compound.

5. A method of identifying a compound modulating the expression and/or biological activity of a GPNMB peptide in a biological sample comprising cells, comprising the steps of
a) contacting a cell expressing a GPNMB peptide with at least one potentially modulating compound, optionally as identified according to any of claims 3 to 5, and
b) measuring a modulation in the expression and/or biological activity of said GPNMB peptide in said cell.

6. The method according to any of claims 3 to 5, wherein contacting said protein with at least one potentially interacting compound is performed in the presence of at least one cofactor.

7. The method according to any of claims 1 to 6, wherein said biological sample is selected from tissues or cells of the heart muscle, such as, for example, cardiac ischemic tissue or cells from the myocardial infarct region (MI) and/or the peri-infarct region (PI).

8. The method according to any of claims 3 to 7, wherein said compound as identified is an inhibitor of the expression and/or biological activity of said GPNMB peptide in said cell and/or tissue.

9. The method according to any of claims 3 to 8, wherein said compound is selected from a peptide library, a combinatory library, a cell extract, in particular a plant cell e x-tract, a "small molecular drug", an antibody or fragment thereof, an antisense oligonucleotide, an siRNA, a protein, and/or a protein fragment.

10. The method according to claim 1 or 2, wherein said method is for monitoring the effect of therapy administered to a subject having a cardiovascular condition.

11. The method according to any claims 1 to 10, wherein said subject is a mammal, such as, for example, a mouse, rat, dog, cat, monkey, rabbit, or human.

12. A method for manufacturing a pharmaceutical composition for treating or preventing a cardiovascular disease, comprising the steps of:
a) performing a method according to any of claims 3 to 11, and
b) formulating said modulator into a pharmaceutical composition.

13. A pharmaceutical composition for treating or preventing a cardiovascular disease, obtainable by a method according to claim 12, preferably comprising a therapeutically effective amount of a compound that downregulates the expression and/or biological activity of GPNMB, and a pharmaceutically acceptable carrier.

14. Use of a modulator of the expression and/or the biological activity of GPNMB in a cell for the manufacture of a pharmaceutical composition for treating or preventing a cardiovascular disease.

15. Use according to claim 14, wherein said cardiovascular disease is selected from the group consisting of atherosclerosis, coronary artery disease, ischemic heart disease, myocardial infarction, chronic heart insufficiency, angina, stroke and other related conditions related to or resulting from an ischemic event.
